(19) Europäisches Patentamt

European Patent Office

Office européen des brevets

(11) Veröffentlichungsnummer : **0 381 304 B1**

(12) **EUROPÄISCHE PATENTSCHRIFT**

(45) Veröffentlichungstag der Patentschrift :
22.09.93 Patentblatt 93/38

(51) Int. Cl.$^5$ : **A61K 31/725**

(21) Anmeldenummer : **90250013.1**

(22) Anmeldetag : **17.01.90**

(54) **Verwendung von Dextransulfat, gegebenenfalls in Kombination mit anti-androgenen Mitteln zur Behandlung des humanen Protatakarzinoms.**

(30) Priorität : **20.01.89 DE 3902021**
**20.09.89 DE 3935855**

(43) Veröffentlichungstag der Anmeldung :
**08.08.90 Patentblatt 90/32**

(45) Bekanntmachung des Hinweises auf die Patenterteilung :
**22.09.93 Patentblatt 93/38**

(84) Benannte Vertragsstaaten :
**AT BE CH DE DK ES FR GB GR IT LI LU NL SE**

(56) Entgegenhaltungen :
**DIALOG INFORMATION SERVICES, Datenbank 159: Cancerlit 1963-90, Zuganspruch 0067437; T. MATSUMOTO et al.: "A case raport of multiple primary carcinomas with marked effects of chemotherapy against oat cell carcinoma"**
**DIALOG INFORMATION SERVICES, Databank 159: Caucerlit 1963-90, Zuganspruch Nr. 0056075; H. MISHINA et al.: "Three cases of pulmonary metastatic adenocarcinoma treated with FT-207 and endoxan"**
**J. CANCER RES. CLIN. ONCOL., Band 105, Nr. 2, 1983, Seiten 189-190, Springer-Verlag; K. ZIMMERMANN et al.: "Inhibition of hyaluronidase by dextransulfate and its possible application in anticancer treatment"**
**INT. J. CANCER, Band 40, 1987, Seiten 511-518, Alan R. Liss, Inc; C.R. PARISH et al.: "Evidence that sulphated polysaccharides inhibit tumour metastasis by blocking tumourcell-derived heparanases"**
**BIOCHIMICA ET BIOPHYSICA ACTA, Band 359, 1974, Seiten 112-129, Elsevier Scientific Publishing Co., Amsterdam, NL; M. KATSUMATA et al: "Separation of multiple dihydrotestosterone receptors in rat ventral prostate by a novel micromethod of electrofocusing. Blocking action of cyproterone acetate and uptake by nuclear chromatin"**

(56) Entgegenhaltungen :
**PROCEEDINGS OF THE SOCIETY FOR EXPERIMENTAL BIOLOGY AND MEDICINE, Band 189, 1988, Seiten 304-309, Society for Experimental Bilology and Medicine; R.S. CHANG et al.: "Dextran sulfate as an inhibitor against the human immunodeficiency virus (42811)"**
**CANCER LETTERS, Band 18, 1983, Seiten 29-34, Elsevier Scientific Publishers Ireland Ltd., IE; I. HIRONO et al.: "Carcinogenicity of dextran sulfate sodium in relation to its molecular weight"**
**J. STEROID BIOCHEM., Band 23, 1985, Seiten 833-841; F. LARRIE et al: "Combination therapy with flutamide and castration (LHRH agonist or orchiectomy)in advanced prostate cancer: a marked improvement in response and survival"**
**"The Merck Manual of Diagnosis and Therapy", 15. Ausgabe, 1987, Seiten 1649-1651, Merck & Co., Inc., Rahway, N.J.**

(73) Patentinhaber : **EnTec Gesellschaft für endokrinologische Technologie m.b.H.**
**Grandweg 64**
**D-22529 Hamburg (DE)**

(72) Erfinder : **Voigt, Klaus-Dieter, Prof. Dr.**
**Horstlooge 22a**
**D-2000 Hamburg 67 (DE)**
Erfinder : **Knabbe, Cornelius, Dr.**
**Diekkamp 32**
**D-2000 Hamburg 67 (DE)**

(74) Vertreter : **UEXKÜLL & STOLBERG**
**Patentanwälte**
**Beselerstrasse 4**
**D-22607 Hamburg (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 381 304 B1

**Beschreibung**

Das Prostatakarzinom (PCA) spielt eine wesentliche Rolle in der Krebsstatistik westlicher Länder. Allein in der Bundesrepublik gibt es in jedem Jahr ca. 8000 Todesfälle. Die Inzidenz mit ca. 10% aller maligner Erkrankungen bei Männern macht das PCA zum zweithäufigsten Tumor des männlichen Geschlechts. So wurden 1987 in den USA 96 000 Neuerkrankungen und 26 000 Todesfälle registriert.

Trotz intensiver Bemühungen in Forschung und klinischer Behandlung ist die altersstandardisierte Mortalitätsrate von 1950 bis 1980 beispielsweise in den USA weitgehend konstant geblieben, während in kleineren europäischen Kollektiven eine deutliche Zunahme beobachtet worden ist, die wegen der höheren Inzidenz in höherem Alter in Zusammenhang mit einer Verschiebung der Altersstruktur der Bevölkerung gesehen werden muß.

Die Androgenabhängigkeit des Prostatakarzinoms ist in der wissenschaftlichen Literatur seit den Untersuchungen von Huggins und Hodges (vgl. Cancer Res. 1, 293-297 (1941)) bekannt.

Im Anschluß an diese Arbeiten wurde eine endokrine Behandlung des Prostatakarzinoms vorgeschlagen, wobei gezeigt werden konnte, daß durch Androgenablation eine Tumorregression erreicht werden kann (vgl. Emmett et a., J. Urol. 83, 471-484 (1960)). Neben der lokalen Verdrängung des Androgens von seinen zellulären Rezeptoren durch Antiandrogene wie Flutamid oder Cyproteronacetat ist die unmittelbare Suppression zirkulierender Androgene durch chirurgische Maßnahmen wie Orchiektomie, Hypophysektomie oder Adrenalektomie oder durch die Verabreichung von Substanzen wie Diethylstilbestrol oder Aminogluthetimid daher bei der Behandlung des fortgeschrittnen Prostatakarzinoms üblich geworden.

Diese endokrine Behandlung führt in 60 bis 80% der Fälle zu einem subjektiven Erfolg. Eine objektive Verbesserung findet allerdings nur bei 20 bis 40% der Patienten statt. Zusätzlich kann eine deutliche Verängerung des symptomfreien Intervalls erreicht werden.

Allerdings führt die androgenablative Therapie nicht zu einer Heilung des Patienten. Die Mehrheit der Patienten verstirbt nach einer anfänglichen Remissionsperiode in der Regel nach dem fast obligaten Übergang des Tumors vom anfänglichen hormonabhängigen in den hormonunabhängigen Zustand.

Die Ätiologie dieses Übergangs des Tumors in den androgenunabhängigen Zustand ist gegenwärtig nicht eindeutig geklärt, wenngleich neuere Unterschungen auf eine initiale Heterogenität des Tumors hinweisen. In dem Tumor lassen sich nämlich sowohl androgenabhängige als auch androgenunabhängige Zellen nachweisen.

Auch das kürzlich beschriebene Therapieschema mit Gonadotropin-Releasing-Hormon-Analoga (GnRH-Analoga) wie z.B. Leuprolid (vgl. Schally et al., Int. J. Gynaecol. Obstet. 18, 318-324 (1989)) beruht auf einer Senkung des peripheren Androgen-Spiegels durch Hemmung der Hypophysen-Gonaden-Achse. Der Hauptvorteil dieser Therapieform liegt gegenüber der konventionellen Behandlung in geringeren Nebenwirkungen, während eine objektive Überlegenheit beispielsweise gegenüber der Therapie mit Diethylstilbestrol nicht nachweisbar war (vgl. u.a. Tolis, G. et al., Proc. Nat. Acad. Scien. 79, 1658-1662 (1982)).

Schließlich hat auch die von Labrie propagierte "komplette" Androgenablation durch Kombination von GnRH-Analoga mit nichtsteroidalen Antiandrogenen bislang weder auf experimenteller Ebene noch in klinischen Studien eine eindeutige Verbesserung gebracht (vgl. Labrie et al., J. Steroid. Biochem. 23, 833-841 (1985)).

Zusammenfassend läßt sich demgemäß feststellen, daß keiner der neueren Therapiepläne zu einer signifikanten Verbesserung der Remissions- und Überlebensrate gegenüber der klassischen endokrinologischen Therapie geführt hat.

Aufgabe der Erfindung ist es demgemäß, Mittel zur Behandlung des humanen Prostatakarzinoms zu schaffen, die gleichzeitig die androgenabhängigen und die androgenunabhängigen Zellen erfassen und damit zur Verbesserung der objektiven Remissionsrate und der Überlebenszeit des betroffenen Patienten führen.

Zur Lösung der Aufgabe wird die Verwendung gemäß Anspruch 1 vorgeschlagen. Die Ansprüche 2 bis 5 betreffen besondere Ausführungsformen der Erfindung.

Überraschenderweise hat es sich erfindungsgemäß gezeigt, daß Dextransulfate in der Lage sind, die Proliferation sowohl androgenabhängiger als auch androgenunabhängiger humaner Prostatakarzinom-Zellen zu hemmen.

Dextransulfate sind Schwefelsäureester des Dextrans, deren Herstellung dem Fachmann bekannt ist. Vorzugsweise liegen die erfindungsgemäß verwendeten Dextransulfate als Natriumsalze vor und weisen mittlere Molekulargewichte von etwa 5000 bis 500 000 auf. In besonders bevorzugter Weise werden erfindungsgemäß Dextransulfate mit mittleren Molekulargewichten zwischen 5000 und 60 000 eingesetzt.

Überraschenderweise sind diese Substanzen in der Lage, sowohl androgenabhängige humane Prostatakarzinom-Zellinien (LNCaP-Zellen) als auch androgenunabhängige Prostatakarzinoms-Zellinien (DU-145) zu hemmen (vgl. Tabelle 2). Die in Tabelle 2 wiedergegebenen Untersuchungsergebnisse wurden unter Einsatz

2

von Dextransulfat mit einem mittleren Molekulargewicht von 5 000 erhalten. Vergleichbare Ergebnisse wurden mit Dextransulfaten anderer mittlerer Molekulargewichts (z.B. 8000 und 500 000) erzielt.

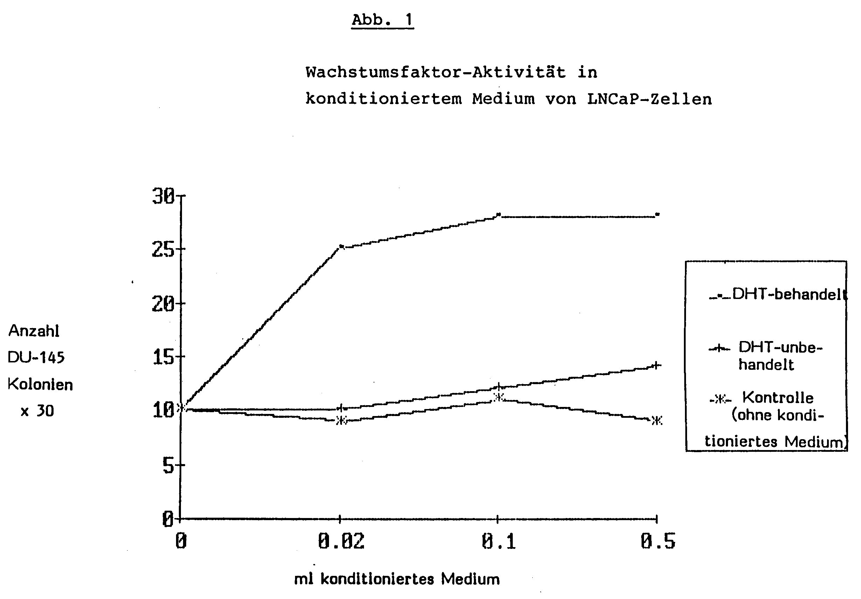

In diesem Zusammenhang konnte erfindungsgemäß erstmals gezeigt werden, daß die androgene Wachstumskontrolle des humanen Prostatakarzinoms durch die Regulation autokriner Wachstumsfaktoren vermittelt wird (Abb. 1). Ferner konnte gezeigt werden, daß diese Regulation in Gegenwart androgenabhängiger Zellen überraschenderweise auch androgenunabhängige Zellen erfaßt (vgl. Tabelle 1), und daß das Wachstum beider Zelltypen durch Dextransulfate gehemmt werden kann (vgl. Tabelle 2).

Oberraschenderweise hat es sich jedoch ferner gezeigt, daß die androgenabhängigen Karzinomzellen zusätzlich über einen Wachstumsfaktor-unabhängigen Regelkreis verfügen (vgl. Tabelle 3),der mit Dextransulfaten nicht zu beeinflussen ist. Während nämlich das Wachstum dieser Zellen in vitro in Abwesenheit von Androgenen nahezu vollständig inhibiert wird, können selbst hohe Dosierungen von Dextransulfat deren Proliferation in Gegenwart von Androgenen (5-$\alpha$-dihydro-Testosteron) nicht in diesem Umfang hemmen.

Erfindungsgemäß werden daher bei gleichzeitigem Vorliegen androgenabhängiger und androgenunabhängiger Zellen pharmazeutische Formulierungen vorgeschlagen, die eine Rombination aus Dextransulfaten und antiandrogenen Mitteln enthalten. Als letztere kommen alle mit Dextransulfat kombinierbaren und vorzugsweise zu fixen Rombinationen verarbeitbaren Mittel in Betracht. Vorzugsweise handelt es sich um Antiandrogene im engeren Sinne, wie beispielsweise Cyproteronacetat oder Flutamid, Hemmer der hypophysären Gonadotropinsekretion, wie beispielsweise LH-RH-Agonisten oder -Antagonisten sowie Inhibitoren der steroidmetabolisierenden Enzyme, wie beispielsweise Inhibitoren der 5$\alpha$-Reduktase oder Aromatase, wobei 5-$\alpha$-Reduktase-inhibitoren besonders bevorzugt sind. Es können auch mehrere antiandrogene Mittel in einer Formulierung oder Packung zusammen mit dem Dextransulfat eingesetzt werden.

Die vorgenannten antiandrogenen Mittel können in fixer Kombination oder getrennt formuliert in einer gemeinsamen Packung mit den Dextransulfaten vorliegen. Ihre Dosierung kann beispielsweise der üblichen und dem Fachmann bekannten Dosierung bei alleiniger Verabreichung entsprechen oder darunter liegen.

Zur Behandlung der hormonunabhängigen Form des Prostatakarzinoms können hingegen Präparate verwendet werden, die Dextransulfat allein enthalten, wobei Dosismengen von 2 bis 40 mg/kg/Tag sowohl in den Kombinationspräparaten als auch in Dextransulfat-Einzelpräparaten in Betracht kommen, jedoch abhängig von der klinischen Gesamtsituation des Patienten darüber oder darunter liegen können.

Die erfindungsgemäßen Präparate können lokal, oral, parenteral, intraperitoneal oder rectal verabreicht werden. Erfindungsgemäß ist die parenterale Verabreichung bevorzugt.

Bei der Herstellung der jeweiligen Formulierungen können neben Dextransulfat und gegebenenfalls den antiandrogenen Mitteln übliche, pharmazeutisch verträgliche Träger- und Zusatzstoffe verwendet und auf dem Fachmann bekannte Weise für die jeweils gewünschte Applikationsform zubereitet werden. Im Einzelfall kann die Bestimmung der jeweils optimalen Dosierung und Applikationsform der Wirkstoffe durch den Fachmann aufgrund seines Fachwissens leicht erfolgen.

Die Erfindung wird nachfolgend anhand von Beispielen erläutert. In den nachfolgenden Beispielen wurden die androgenabhängige humane PCA-Zellinie LNCaP (vgl. Horoszewicz et al., Cancer Research 43, 1809-1818 (1983)), sowie die androgenunabhängige humane Prostatakarzinom-Zellinie DU-145 ATCC Nr. HTB81 verwendet und unter Standardbedingungen in DMEM-Kulturmedium angereichert mit 10% fetalem Kälberserum als Dauerkultur gezogen.

Für die jeweiligen Wachstumsversuche wurde das Kulturmedium weitgehend von endogenen Steroiden befreit (vgl. Darbre et a., Cancer Res. 43, 349-354 (1983)).

### Beispiel 1

Es wurde der Einfluß von 5-$\alpha$-Dihydro-Testosteron allein sowie in Gegenwart von LNCaP-Zellen auf DU-145-Zellen untersucht.

Der Versuch wurde in einer 4-kammerigen Petrischale durchgeführt, die zuvor mit 0,1 mg/ml Poly-D-Lysin benetzt worden war.

Je 1000 DU-145 Zellen wurden in zwei Kammern ausgesät. Die Zellen in einer der Rammern wurden mit DHT in einer Endkonzentration $10^{-8}$ M in ethanolischer Lösung behandelt; während die Zellen in der zweiten Kammer zur Kontrolle nur mit dem Lösungsmittel behandelt wurden. Die Ethanol-Endkonzentration betrug in beiden Ansätzen 0,1%.

Als Wachstumsindikator wurde die nach Ablauf von 5 Tagen erreichte Anzahl der DU-145 Kolonien mit mehr als 50 Zellen gewählt, die nach Fixierung mit Methanol und Färbung mit Amidoschwarz mikroskopisch bestimmt wurde.

In einem weiteren Ansatz wurden zwei Kammern der Petrischale mit je 5 x $10^5$ LNCaP-Zellen besät und die Zellen in einer der Kammern wie oben angegeben mit DHT in ethanolischer Lösung stimuliert, während die zweite Kammer wiederum als Kontrolle diente.

Die beiden übrigen Kammern wurden wie oben angegeben jedoch ohne weitere Zusätze mit DU-145 Zellen besät.

Nach 24 Stunden wurde die Trennwand jeweils zwischen einer mit DU-135 Zellen besäten Kammer und einer mit LNCaP-Zellen besäten Kammer perforiert, so daß immer eine DU-145-Kammer mit einer LNCaP-Kammer über das Medium in Verbindung treten konnte. Die Wachstumsraten in den jeweiligen Kammern wurden erneut bestimmt.

Die Ergebnisse der beiden Ansätze sind in Tabelle 1 wiedergegeben.

## Tabelle 1

## Wachstum von DU-145 in Kokultur mit LNCaP

|  | Behandlung mit Ethanol (=Kontrolle) | Behandlung mit DHT |
|---|---|---|
| keine LNCaP in Kokultur (=Kontrolle) | 107 | 104 |
| LNCaP in Kokultur | 136 | 213 |

Die Ergebnisse zeigen, daß DHT allein erwartungsgemäß ohne Einfluß auf die DU-145-Zellen ist, während in Gegenwart von LNCaP-Zellen ohne androgene Stimulation eine leichte Wachstumssteigerung zubeobachten ist, die sich bei androgener Stimulation erheblich steigert.

Diese Ergebnisse lassen darauf schließen, daß die unter dem Einfluß von DHT von den LNCaP-Zellen gebildete Aktivität das Wachstum auch der androgenunabhängigen Zellen anregt.

## Biespiel 2

In einem weiteren Versuch wurde serumfreies, konditioniertes Medium von LNCaP-Zellen mit und ohne androgene Stimulation nach einem Verfahren, modifiziert nach Dickson et al., Endocrinology, 118, 138-142 (1986)) gewonnen.

Das konditionierte Medium wurde zur Prüfung seiner Wachstumsfaktor-Aktivität im Soft-agar-assay mit DU-145 Zellen nach dem Verfahren wie nachfolgend in Beispiel 3 erläutert untersucht.

Die Ergebnisse sind in Abb. 1 wiedergegeben. Sie bestätigen die Induktion von Wachstumsfaktor-Aktivitäten im Medium von LNCaP-Zellen durch Androgene.

Die peptidische Natur der Wachstumsfaktor-Aktivitäten wurde durch Protease- und Hitzeeinaktivierung nachgewiesen.

## Biespiel 3

Die das Wachstum beider Zelltypen hemmende Wirkung von Dextransulfat (MG 5000, Sigma D0768) wurde im Soft-agar-assay nachgewiesen (modifiziert nach Hamburger und Salmon, Science 197, 461 (1977)).

0,8 ml einer oberen Schicht bestehend aus DMEM, 0,4% Bacto-agar, 10% fetalem Kälberserum, sowie der zu testenden Probe ($10^{-8}$) M DHZ bzw. Ethanol und/oder Dextransulfat) und den Indikatorzellen (15.000 DU-145 oder LNCaP-Zellen) wurde in 35 mm Schalen auf eine schon ausgehärtete untere Schicht aus 1,0 ml DMEM mit 0,6% Agar und 10% fetalem Kälberserum gegeben. Die Schalen wurden über 14 Tage bei 37°C und 5% Kohlendioxid Spannung inkubiert. Als Wachstumsparameter wurden mikroskopisch Zellkolonien größer als 50 μm gezählt.

Die Ergebnisse sind in den Tabellen 2 und 3 wiedergegeben.

### Tabelle 2

### Wachstum von LNCaP- und DU-145-Zellen

| | -- | Dextran-sulfat 10 μg/ml | Dextran-sulfat 100 μg/ml | Dextran-sulfat 1000 μg/ml |
|---|---|---|---|---|
| DU-145 | 380 | 350 | 125 | 60 |
| LNCaP | 480 | 360 | 50 | 50 |

### Tabelle 3

### Wachstum von LNCaP-Zellen

| | -- | Dextran-sulfat 10 μg/ml | Dextran-sulfat 100 μg/ml | Dextran-sulfat 1000 μg/ml |
|---|---|---|---|---|
| Ethanol | 480 | 360 | 50 | 50 |
| + $10^{-8}$ M DHT | 900 | 560 | 240 | 200 |

Wie Tabelle 2 zeigt, werden sowohl LNCaP-Zellen als auch DU-145 Zellen durch Dextransulfat bei einer Konzentration von 100 μg/ml in ihrem Wachstum deutlich gehemmt.

Im Gegensatz dazu geht aus Tabelle 3 hervor, daß selbst Dextransulfat-Konzentrationen von 1000 μg/ml in Gegenwart von DHT die Proliferation von LNCaP-Zellen nur unvollständig hemmen. Dies läßt darauf schließen, daß die Zellen über einen Dextransulfatresistenten Regelkreis verfügen, dessen biologische Wertigkeit und biochemische Identität noch ungeklärt sind.

Maximale Hemmung von LNCaP-Zellen ist demgemäß nur durch Gabe von Dextransulfat in Abwesenheit von DHT (Androgenentzug) möglich.

Analoge Ergebnisse wurden mit Dextransulfat MG 8000 (Sigma D6393) und MG 500 000 (Sigma D6001) erhalten.

**Patentansprüche**

1. Verwendung von Dextransulfaten als Wirkstoffe zur Herstellung pharmazeutischer Präparate zur Behandlung des humanen Prostatakarzinoms.

2. Verwendung nach Anspruch 1 in Verbindung mit anti-androgenen Mitteln.

3. Verwendung nach Anspruch 2, dadurch gekennzeichnet, daß die pharmazeutischen Präparate als fixe Kombinationen aus Dextransulfat und einem oder mehreren antiandrogenen Mitteln vorliegen.

4. Verwendung nach Anspruch 2, dadurch gekennzeichnet, daß die pharmazeutischen Präparate als Packungen vorliegen, in denen das Dextransulfat und das/die androgene(n) Mittel getrennt formuliert vorhanden sind.

5. Verwendung nach den Ansprüchen 2 bis 4, dadurch gekennzeichnet, daß als antiandrogene Mittel Cyproteronacetat, Flutamid, LH-RH-Agonisten, LH-RH-Antagonisten, Inhibitoren der 5α-Reduktase oder Inhibitoren der Aromatase verwendet werden.

6. Verwendung nach den Ansprüchen 1 bis 5, dadurch gekennzeichnet, daß das mittlere Molekulargewicht des Dextransulfats 5000 bis 60 000 beträgt.

**Claims**

1. Use of dextran sulphates as active ingredients for producing pharmaceutical mixtures for the treatment of human prostatic carcinoma.

2. Use according to claim 1 in combination with antiandrogenic agents.

3. Use according to claim 2, characterised in that the pharmaceutical mixtures are present as fixed combinations of dextran sulphate and one or more antiandrogenic agents.

4. Use according to claim 2, characterised in that the pharmaceutical mixtures exist as packs in which the dextran sulphate and the androgenic agent(s) are present as separately formulated ingredients.

5. Use according to claims 2 to 4, characterised in that cyproterone acetate, flutamide, LH-RH antagonists, 5-α-reductase or aromatase inhibitors are used as antiandrogenic agents.

6. Use according to claims 1 to 5, characterised in that the average molecular weight of dextran sulphate is 5 000 to 60 000.

**Revendications**

1. Utilisation de sulfates de dextrane en tant qu'agents actifs pour la production de préparations pharmaceutiques pour le traitement du carcinome humain de la prostate.

2. Utilisation selon la revendication 1, en liaison avec des agents anti-androgènes.

3. Utilisation selon la revendication 2, caractérisée en ce que les préparations pharmaceutiques sont sous la forme de combinaisons fixes de sulfate de dextrane avec un ou plusieurs agents anti-androgènes.

4. Utilisation selon la revendication 2, caractérisée en ce que les préparations pharmaceutiques sont sous la forme de paquets dans lesquels sont formulés séparément le sulfate de dextrane et le ou les agents androgènes.

5. Utilisation selon les revendication 2 à 4, caractérisée en ce qu'on utilise, en tant qu'agents antiandrogènes, l'acétate de cyprotérone, le flutamide, des agonistes de LH-RH, des antagonistes de LH-RH, des inhibiteurs de la 5α-réductase ou bien des inhibiteurs de l'aromatase.

6. Utilisation selon les revendications 1 à 5, caractérisée en ce que le poids moléculaire moyen du sulfate de dextrane est compris entre 5.000 et 60.000.

Abb. 1

Wachstumsfaktor-Aktivität in
konditioniertem Medium von LNCaP-Zellen